# FASCICULE DE BREVET EUROPEEN

(11) **EP 2 895 448 B1**
(45) Date de publication et mention de la délivrance du brevet: **06.07.2016**
(21) Numéro de dépôt: 13766124.5
(22) Date de dépôt: 06.09.2013
(51) Int. Cl.: C07C 9/14, C07C 1/24, C07C 2/10, C07C 2/24, C07C 5/03, C10G 50/00, C10G 45/00

(54) **PROCÉDÉ DE PRODUCTION DE KÉROSÈNE À PARTIR DE BUTANOLS**
VERFAHREN ZUR HERSTELLUNG VON KEROSIN AUS BUTANOLEN
METHOD FOR PRODUCING KEROSENE FROM BUTANOLS

(30) Priorité: 12.09.2012 FR 1202429
(43) Date de publication de la demande: 22.07.2015
(73) Titulaire: IFP Énergies nouvelles, 92852 Rueil-Malmaison Cedex (FR)
(72) Inventeur: VIVIEN, Tom, F-69007 Lyon (FR); COUPARD, Vincent, F-69100 Villeurbanne (FR); HUGUES, Francois, F-69390 Vernaison (FR); HUYGHE, Raphael, F-69700 Saint Andeol Le Chateau (FR)
(86) Numéro de dépôt international: PCT/FR2013/052054
(87) Numéro de publication internationale: WO 2014/041285

(56) Documents cités:
- WO-A1-2009/145861
- WO-A2-2009/079213
- US-A1- 2006 135 832
- US-B2- 7 572 946

## Description

### DOMAINE TECHNIQUE DE L'INVENTION

La présente invention concerne la transformation de butanol, et plus particulièrement de biobutanol, en base carburant.

Elle concerne plus particulièrement un procédé catalytique flexible de transformation du butanol en distillats moyens.

### ART ANTÉRIEUR

La demande pour une utilisation de ressources renouvelables en remplacement partiel des ressources pétrolières pour la synthèse de carburants va croissante. Ainsi l'utilisation de biobutanol dans la synthèse de bases pour carburants reçoit un intérêt de plus en plus marqué.

Par biobutanol, on entend un butanol produit à partir de ressources renouvelables issues de la biomasse telles que par exemple les matières premières ligno-cellulosiques.

Ces dernières sont des matières cellulosiques, c'est-à-dire constituées à plus de 90% poids de cellulose, et/ou lignocellulosiques, c'est-à-dire constituées de cellulose, d'hémicelluloses, qui sont des polysaccharides essentiellement constitués de pentoses et d'hexoses ainsi que de lignine, qui est une macromolécule de structure complexe et de haut poids moléculaire, composée d'alcools aromatiques reliés par des liaisons éther.

L'émergence des projets de production de biobutanol est largement portée par les volontés publiques et industrielles de développement de biocarburants de seconde génération, valorisant à terme un vaste panel de matières premières ligno-cellulosiques.

Par comparaison avec l'éthanol, le n-butanol et l'isobutanol présentent plusieurs avantages : plus forte densité énergétique; caractère moins hydrophiles; meilleures compatibilités, tant avec les infrastructures de stockage/transport des carburants qu'avec les moteurs automobiles courants; pressions de vapeur moins élevées; moins corrosifs. L'isobutanol est particulièrement compatible avec le carburant diesel alors que le n-butanol est miscible avec l'essence.

Le n-butanol est un des produits des fermentations dites ABE (acétone-butanol-éthanol) de Clostridium acetobutylicum. Les projets récents utilisent des souches sélectionnées, mutantes ou OGM permettant une production spécifique de n-butanol, un des facteurs limitant étant sa toxicité pour les microorganismes concernés. Des souches plus tolérantes et des procédés d'extraction en continu ou semi-continu sont donc nécessaires. Les bactéries utilisées dans ces procédés sont toujours du genre Clostridium.

L'isobutanol, ou 2-méthylpropane-1-ol, est un isomère ramifié du n-butanol. Il est également un produit de la fermentation des glucides. Ses voies de biosynthèse sont toutefois différentes de celles du n-butanol. Les procédés de production de l'isobutanol sont en cours de développement par des acteurs comme Gevo ou Butamax à partir de sources bactériennes différentes de celles utilisées pour la production du n-butanol (E.Coli).

La demande de brevet WO2009/079213 décrit la production de bases pour carburants à partir de biomasse comprenant des étapes de fermentation en alcool, de déshydratation d'une partie desdits alcools en oléfines, d'oligomérisation d'une partie desdites oléfines et d'hydrogénation éventuelle du produit de l'oligomérisation. En revanche, le brevet ne propose pas de moyen de contrôler l'augmentation de température dans le réacteur due à l'exothermicité de la réaction d'oligomérisation. L'oligomérisation est réalisée en une seule étape.

La demande de brevet WO2011/140560 décrit la production d'une base kérosène à partir de matières premières ligno-cellulosiques en passant par une voie isobutanol. Lesdites matières premières sont fermentées dans des conditions spécifiques propres à la production de l'isobutanol. Celui-ci est ensuite déshydraté puis oligomérisé pour produire une coupe proche du kérosène. Cette demande n'adresse pas le problème du contrôle de l'augmentation de température dans le réacteur due à l'exothermicité de l'oligomérisation, ni le contrôle de la composition des produits obtenus.

### OBJET ET INTÉRÊT DE L'INVENTION

La présente invention a trait à un procédé de production de bases hydrocarbonées distillats moyens à partir d'une charge butanol, ledit procédé comprenant au moins :
a) une étape de déshydratation isomérisante de ladite charge butanol en présence d'un catalyseur acide amorphe ou zéolithique dans au moins un réacteur, opérant à une pression absolue en entrée de réacteur comprise entre 0,5 et 1,2 MPa et à une température en entrée de réacteur comprise entre 350 et 450°C de manière à produire un effluent majoritairement butylènique,
b) une étape de séparation de l'eau présente dans ledit effluent butylènique opérant à une pression comprise entre 0,5 et 1,2 MPa et à une température comprise entre 35 et 60°C,
c) une étape de purification de l'effluent liquide organique issu de l'étape b) de manière à produire un effluent organique purifié,
d) une première étape d'oligomérisation d'une charge comprenant au moins une partie de l'effluent organique purifié issu de l'étape c), la totalité de l'effluent issu de l'étape g) et au moins une partie du produit léger issu de l'étape f) en présence d'un catalyseur amorphe dans au moins un réacteur opérant à une pression absolue comprise entre 0,5 et 10 MPa, à une température comprise entre 40 et 110°C et à une vitesse volumique horaire comprise entre 0,1 et 10 h⁻¹, de manière à produire un effluent de première oligomérisation, comprenant au moins 50% poids d'oléfines ayant un nombre d'atomes de carbone supérieur ou égal à 8, le pourcentage poids étant exprimé par rapport à la masse totale des oléfines contenues dans ledit effluent,
e) une deuxième étape d'oligomérisation dudit effluent de première oligomérisation, en présence d'un catalyseur amorphe dans au moins un réacteur opérant à une pression absolue comprise entre 2 et 15 MPa, à une température comprise entre 100 et 200°C et à une vitesse volumique horaire comprise entre 0,1 et 10 h⁻¹, de manière à produire un effluent de deuxième oligomérisation,
f) une étape de fractionnement dudit effluent de deuxième oligomérisation en au moins trois produits correspondants respectivement à un produit léger comprenant majoritairement les composés C₂ à C₄, un produit intermédiaire comprenant majoritairement les composés C₅ à C₉, et un produit distillat moyen comprenant majoritairement les composés ayant au moins 10 atomes de carbone,
g) une troisième étape d'oligomérisation d'au moins une partie dudit produit intermédiaire en présence d'un catalyseur amorphe dans au moins un réacteur opérant à une pression absolue comprise entre 2 et 15 MPa, à une température comprise entre 100 et 200°C et à une vitesse volumique horaire comprise entre 0,1 et 5 h⁻¹,
h) une étape d'hydrogénation d'au moins une partie dudit produit distillat moyen en présence d'un catalyseur comprenant au moins un métal du groupe VIII dans au moins un réacteur opérant à une pression absolue comprise entre 2 et 4 MPa, à une température comprise entre 100 et 350°C et à une vitesse volumique horaire comprise entre 1 et 5 h⁻¹ avec un ratio molaire hydrogène sur hydrocarbures H₂/HC compris entre 10 et 450.

Un avantage de l'invention est un meilleur contrôle des produits obtenus, par la séparation des deux étapes d'oligomérisation, ladite séparation permettant également de limiter l'augmentation de température dans le réacteur due à l'exothermicité des réactions dans chacune desdites étapes.

Un autre avantage de l'invention est que l'on peut ajouter des aromatiques à la base carburant produite conformément à l'invention jusqu'aux limites prévues par la norme ASTM D7566-11 a tout en respectant la spécification de densité de ladite norme.

### DESCRIPTION DÉTAILLÉE DE L'INVENTION

### Charge

La charge traitée dans le procédé selon l'invention est une charge butanol, le terme butanol caractérisant l'ensemble des alcools comprenant 4 atomes de carbone. Ladite charge butanol est avantageusement une charge biobutanol, c'est-à-dire une charge butanol produite à partir de ressources renouvelables issues de la biomasse. Ladite charge butanol comprend majoritairement du butanol, à hauteur de plus de 50% poids et de préférence plus de 70% poids et comprend également de l'eau, à une teneur comprise entre 0 et 50% poids, de préférence entre 0% et 30% poids, et de manière préférée entre 15% et 25% poids, une teneur en impuretés cationiques telles que par exemple les ions Na⁺, Ca²⁺, K⁺, Mn²⁺, Fe²⁺, Cu²⁺, Zn²⁺ avantageusement inférieure à 0,1% poids, une teneur en impuretés anioniques telles que par exemple les ions chlorure, sulfate, nitrite, nitrate, phosphate avantageusement inférieure à 0,1% poids, une teneur en métaux tel que le nickel, le chrome et le potassium avantageusement inférieure à 0,1% poids, une teneur en autres alcools tels que par exemple le méthanol et l'éthanol avantageusement inférieure à 10% poids, et de préférence inférieure à 5% poids, une teneur en composés oxygénés autres que les alcools tels que par exemple les éthers, les acides, les cétones, les aldéhydes, les acétals et les esters avantageusement inférieure à 1% poids et une teneur en composés azotés et en composés soufrés tels que par exemple les amines, les acétonitriles, les sulfates nitriques, le sulfure de carbone avantageusement inférieure à 0,5% poids, les pourcentages poids étant exprimés par rapport à la masse totale de ladite charge butanol.

Le procédé selon l'invention comprend avantageusement une étape de purification effectuée préalablement à l'étape a) de déshydratation de manière à éliminer les impuretés cationiques et anioniques ainsi qu'au moins une partie des composés oxygénés pour limiter la désactivation du catalyseur de déshydratation placé en aval.

Ladite étape de purification est avantageusement mise en oeuvre par des moyens connus de l'homme du métier, tels que par exemple l'utilisation d'au moins une résine, l'adsorption des impuretés et des composés oxygénés sur des solides choisis parmi les tamis moléculaires, le charbon actif, l'alumine et les zéolithes et la distillation pour produire une charge butanol purifiée répondant aux niveaux d'impuretés compatibles avec le catalyseur de déshydratation et un produit comprenant les impuretés organiques.

Lesdits niveaux d'impuretés compatibles avec le catalyseur de déshydratation sont une teneur en impuretés ioniques inférieure à 100 ppm, une teneur en métaux inférieure à 50 ppm, une teneur en impuretés oxygénées inférieure à 150 ppm et une teneur en composés azotés et soufrés inférieure à 30 ppm.

Une étape de prétraitement peut également avantageusement être mise en oeuvre par hydrogénation des composés insaturés oxygénés en présence d'un catalyseur à base de nickel, ladite étape de prétraitement étant mise en oeuvre avant ou après l'étape de purification et de préférence après.

### Étape a) de Déshydratation

Conformément à l'invention, la charge butanol, éventuellement purifiée, subit une étape a) de déshydratation isomérisante de manière à produire un effluent majoritairement butylènique, ladite étape opérant en présence d'un catalyseur de déshydratation connu de l'homme du métier, en particulier d'un catalyseur acide amorphe ou d'un catalyseur acide zéolithique dans au moins un réacteur.

On entend par effluent majoritairement butylènique un effluent comprenant de l'eau et au moins 95%, de préférence au moins 97% et de manière préférée, au moins 98% poids de butènes par rapport à la masse totale des composés carbonés présents dans ledit effluent produit par ladite étape a). Outre la présence majoritaire de butènes, ledit effluent carboné peut également comprendre d'autres composés hydrocarbonés, hydroxycarbonés ou oxycarbonés en une proportion très minoritaire. En particulier, ledit effluent carboné comprend avantageusement moins de 5%, de préférence moins de 3% et de manière préférée moins de 2% poids de composés ayant un nombre d'atomes de carbone supérieur ou égal à 5 et de composés oxygénés tels que par exemples le CO₂, le CO, le diéthyléther et l'acétaldéhyde, les pourcentages étant exprimés en pourcentages poids par rapport à la masse totale des composés carbonés présents dans ledit effluent produit à ladite étape a).

Ladite étape a) de déshydratation isomérisante permet de convertir l'isobutanol en un mélange de butènes.

Dans le cas où le catalyseur utilisé dans l'étape a) de déshydratation est un catalyseur zéolithique, ledit catalyseur comprend au moins une zéolithe choisie parmi les zéolithes ayant au moins des ouvertures de pores contenant 10 ou 12 atomes d'oxygènes (10MR ou 12 MR). Il est connu en effet de définir la taille des pores des zéolithes par le nombre d'atomes d'oxygène formant la section annulaire des canaux des zéolithes, appelés "member ring" ou MR en anglais. De manière préférée, ledit catalyseur zéolithique comprend au moins une zéolithe présentant un type structural choisi parmi les types structuraux MFI, FAU, MOR, FER, et BEA.

La zéolithe mise en oeuvre dans le catalyseur utilisé dans l'étape a) du procédé selon l'invention peut avantageusement être modifiée par désalumination ou désilication selon toute méthode de désalumination ou désilication connue de l'homme du métier.

Dans le cas où le catalyseur utilisé dans l'étape a) de déshydratation est un catalyseur acide amorphe, ledit catalyseur comprend au moins un oxyde réfractaire poreux choisi parmi l'alumine, l'alumine activée par un dépôt d'acide minéral et la silice alumine.

Ledit catalyseur de déshydratation amorphe ou zéolithique utilisé dans l'étape a) du procédé selon l'invention peut également avantageusement comprendre au moins une matrice de type oxyde également appelé liant. On entend par matrice selon l'invention, une matrice amorphe ou mal cristallisée.

Ladite matrice est avantageusement choisie parmi les éléments du groupe formé par les argiles (telles que par exemple parmi les argiles naturelles telles que le kaolin et la bentonite), la magnésie, les alumines, les silices, les silice-alumines, les aluminates, l'oxyde de titane, l'oxyde de bore, la zircone, les phosphates d'aluminium, les phosphates de titane, les phosphates de zirconium, et le charbon. De préférence ladite matrice est choisie parmi les éléments du groupe formé par les alumines, les silices et les argiles.

Dans un mode préféré, le liant possède une texture macroporeuse comme décrit dans le brevet US 7,880,048.

Le catalyseur de déshydratation utilisé dans l'étape a) du procédé selon l'invention est avantageusement mis en forme sous la forme de grains de différentes formes et dimensions. Il est avantageusement utilisé sous la forme d'extrudés cylindriques ou polylobés tels que bilobés, trilobés, polylobés de forme droite ou torsadée, mais peut éventuellement être fabriqué et employé sous la forme de poudre concassée, de tablettes, d'anneaux, de billes, de roues, de sphères. De préférence, ledit catalyseur est sous forme d'extrudés ou de billes.

L'étape a) de déshydratation isomérisante du procédé selon l'invention opère avantageusement à une pression absolue en entrée de réacteur comprise entre 0,5 et 1,2 MPa, préférentiellement entre 0,6 et 1,1 MPa et à une température comprise entre 350 et 450°C. La pression absolue en entrée de réacteur est choisie de manière à ce que ladite charge de ladite étape a) est en phase gaz en entrée de réacteur.

La pression de fonctionnement élevée, c'est-à-dire supérieure à 0,5 MPa, préférentiellement supérieure à 0,6 MPa, permet avantageusement de séparer les produits de l'eau par décantation liquide/liquide. La vitesse volumique horaire est comprise entre 2 et 7 h⁻¹.

Par vitesse volumique horaire, on entend le débit volumique de la charge en entrée de réacteur en m³/h à 15°C, 1 atm divisé par le volume de catalyseur en m³ contenu dans le réacteur.

Ladite étape a) est très endothermique. Elle est donc avantageusement opérée dans au moins deux réacteurs distincts, l'effluent d'un réacteur étant réchauffé avant d'être envoyé comme charge du réacteur suivant. La température à l'entrée de chaque réacteur est comprise entre 350 et 450°C, préférentiellement entre 350 et 400°C, plus préférentiellement entre 350 et 375°C.

La conversion de la charge butanol dans l'étape a) est avantageusement supérieure à 95%, de préférence 99% et de manière préférée supérieure à 99,8%. On entend par conversion de la charge butanol, le rapport de la différence entre le débit massique de la charge butanol en entrée de l'étape a) et le débit massique de la charge butanol en sortie de l'étape a) sur le débit massique de la charge butanol en entrée de l'étape a).

### Étape b) de Séparation de l'eau

Conformément à l'invention, l'effluent majoritairement butylènique issu de l'étape a) subit au moins une étape b) de séparation de l'eau présente dans ledit effluent. L'eau a un effet néfaste sur les catalyseurs des étapes subséquentes du procédé selon l'invention. Les effluents de l'étape b) sont un effluent liquide aqueux, un effluent liquide organique et éventuellement un effluent gazeux comprenant du monoxyde de carbone, du dioxyde de carbone, de l'hydrogène, du méthane et de l'acétone, pris seul ou en mélange. Ladite étape b) est préférentiellement une étape de décantation dans laquelle une phase aqueuse est séparée d'une phase organique.

Ladite étape b) opère avantageusement à une pression comprise entre 0,5 et 1,2 MPa, préférentiellement entre 0,6 et 1,1 MPa et à une température comprise entre 35 et 60°C.

La pression de fonctionnement élevée de l'étape a) de déshydratation selon l'invention permet de condenser l'eau dans l'étape b) à une température élevée. Lorsque l'étape de déshydratation est réalisée à une pression plus faible, il est soit nécessaire d'utiliser un cycle froid pour condenser l'eau, soit nécessaire de comprimer l'effluent en sortie de l'étape de déshydratation.

### Étape c) de Purification

Conformément à l'invention, l'effluent liquide organique issu de l'étape b) subit une étape c) de purification de manière à produire un effluent organique purifié.

Ladite étape c) peut avantageusement être mise en oeuvre par toute méthode connue de l'homme du métier, par exemple par un traitement dans une colonne d'absorption à la MDEA (méthyldiéthylamine) ou autre amine suivi d'un traitement sur tamis moléculaire, lesdits tamis étant avantageusement de type 13X, 3A, 4A et 5A, pris seul ou en mélange. Ladite étape c) peut également avantageusement être mise en oeuvre par traitement dans une colonne de lavage à la soude. Ladite étape c) peut également avantageusement être mise en oeuvre par traitement dudit effluent organique sur tamis moléculaire, lesdits tamis étant avantageusement de type 13X, 3A, 4A et 5A, pris seul ou en mélange. Ladite étape c) peut également avantageusement comprendre un traitement sur résine basique de manière à retirer les alcools présents dans ledit effluent liquide organique. Des sécheurs peuvent avantageusement être mis en oeuvre de manière à atteindre une teneur en eau compatible avec les catalyseurs d'oligomérisation utilisés en aval dans les étapes d) et e) d'oligomérisation.

La teneur en eau dudit effluent organique purifié est avantageusement comprise entre 0 et 1000 ppm, de préférence entre 0 et 500 ppm et de manière préférée entre 0 et 200 ppm. La teneur en constituants soufrés, par exemple l'H₂S ou le COS, dudit effluent organique purifié est avantageusement inférieure à 100 ppm, de préférence inférieure à 50 ppm. La teneur en constituants azotés, par exemple l'ammoniaque, dudit effluent organique purifié est inférieure à 1 ppm. La teneur en constituants inorganiques dudit effluent organique purifié est inférieure à 1 ppm, préférentiellement inférieure aux limites de détection.

### Étape d) d'Oligomérisation sélective des isobutènes dite « Sélectopol »

Conformément à l'invention, au moins une partie de l'effluent organique purifié issu de l'étape c) de purification, la totalité de l'effluent issu de l'étape g) d'oligomérisation et au moins une partie du produit léger issu de l'étape f) de fractionnement sont mélangées de manière à former une charge de première oligomérisation, ladite charge subissant une première étape d) d'oligomérisation sélective des isobutènes dans au moins un réacteur en présence d'un catalyseur amorphe de manière à produire un effluent de première oligomérisation.

Par au moins une partie de l'effluent organique purifié issu de l'étape c), on entend au moins 50 % poids, de préférence au moins 90% poids et de manière préférée la totalité de l'effluent issu de ladite étape c), les pourcentages poids étant exprimés par rapport à la masse totale dudit effluent.

La conversion de l'isobutène dans l'étape d) est avantageusement supérieure à 80%, de préférence supérieure à 85% et de manière préférée supérieure à 90%. On entend par conversion de l'isobutène, le rapport de la différence entre le débit massique d'isobutène en entrée de l'étape d) et le débit massique d'isobutène en sortie de l'étape d) sur le débit massique d'isobutène en entrée de l'étape d).

La conversion des n-butènes dans l'étape d) est avantageusement inférieure à 15%, de préférence inférieure à 10% et de manière préférée inférieure à 5%. La conversion des n-butènes est définie de manière analogue à la conversion de l'isobutène.

Ledit effluent de première oligomérisation comprend au moins 50% poids d'oléfines ayant un nombre d'atomes de carbone supérieur ou égal à 8, le pourcentage poids étant exprimé par rapport à la masse totale des oléfines contenues dans ledit effluent. En particulier, ledit effluent comprend au moins 60% poids, de préférence au moins 65% et de manière préférée, au moins 75% poids, d'oléfines ayant un nombre d'atomes de carbone supérieur ou égal à 8, le pourcentage poids étant exprimé par rapport à la masse totale des oléfines contenues dans ledit effluent. Parmi les oléfines ayant un nombre d'atomes de carbone supérieur ou égal à 8, les oléfines en C₈-C₁₂ sont majoritaires par rapport aux oléfines ayant au moins 13 atomes de carbone, c'est-à-dire que le rapport massique des oléfines en C₈-C₁₂ sur les oléfines ayant au moins 13 atomes de carbone est supérieur à 1.

Outre la présence majoritaire d'oléfines ayant un nombre d'atomes de carbone supérieur ou égal à 8, l'effluent oléfinique produit lors de la première étape d'oligomérisation d) comprend également avantageusement moins de 50%, de préférence moins de 40% et de manière préférée, moins de 35% et de manière très préférée, moins de 25% poids de composés oléfiniques dont le nombre d'atomes de carbone est inférieur ou égal à 4, les pourcentages poids étant exprimés par rapport à la masse totale des oléfines contenues dans l'effluent produit.

Le recyclage de la totalité de l'effluent issu de l'étape g) d'oligomérisation et d'au moins une partie du produit léger issu de l'étape f) de fractionnement permet d'améliorer le rendement de conversion des oléfines ayant moins de 8 atomes de carbone par un meilleur contrôle de l'exothermicité.

Les oléfines comprenant au moins 16 atomes de carbone réagissent très peu dans l'étape d) et dans l'étape e). Leur recyclage en amont de l'étape d) permet de minimiser l'élévation de température, que ce soit dans la section réactionnelle de l'étape d) ou dans la section réactionnelle de l'étape e). Ce recyclage permet donc un meilleur contrôle de température dans lesdites sections réactionnelles.

Le catalyseur mis en oeuvre dans la première étape d'oligomérisation d) comprend au moins un élément du groupe VIII de préférence choisi parmi le nickel, le cobalt, le fer, le platine et le palladium et de manière préférée, ledit élément est le nickel et au moins un support réfractaire oxyde poreux choisi de préférence parmi l'alumine, la silice, les silices-alumines, les alumines silicées, les zircones, l'oxyde de titane, la magnésie, les argiles pris seuls ou en mélange et de manière préférée, ledit support est une alumine ou une silice alumine, préférentiellement une silice-alumine. Dans un arrangement préféré, le catalyseur mis en oeuvre dans la première étape d'oligomérisation d) est un catalyseur à base de silice-alumine tel que décrit dans le brevet US 7,572,946.

Ledit catalyseur utilisé dans ladite étape d) du procédé selon l'invention comprend également avantageusement au moins une matrice de type oxyde également appelé liant. On entend par matrice selon l'invention, une matrice amorphe ou mal cristallisée.

Ladite matrice est avantageusement choisie parmi les éléments du groupe formé par les argiles (telles que par exemple parmi les argiles naturelles telles que le kaolin ou la bentonite), la magnésie, les alumines, les silices, les silice-alumines, les aluminates, l'oxyde de titane, l'oxyde de bore, la zircone, les phosphates d'aluminium, les phosphates de titane, les phosphates de zirconium, et le charbon. De préférence ladite matrice est choisie parmi les éléments du groupe formé par les alumines, les argiles et les silices, de manière plus préférée ladite matrice est choisie parmi les alumines, et de manière encore plus préférée ladite matrice est l'alumine gamma.

Le catalyseur utilisé dans ladite étape d) du procédé selon l'invention est avantageusement mis en forme sous la forme de grains de différentes formes et dimensions. Il est avantageusement utilisé sous la forme d'extrudés cylindriques ou polylobés tels que bilobés, trilobés, polylobés de forme droite ou torsadée, mais peut éventuellement être fabriqué et employé sous la forme de poudre concassée, de tablettes, d'anneaux, de billes, de roues, de sphères. De préférence, ledit catalyseur est sous forme d'extrudés de taille comprise entre 1 et 10 mm.

Ladite première étape d'oligomérisation d) du procédé selon l'invention opère avantageusement à une température comprise entre 40 et 110°C, de préférence entre 60 et 90°C, à une pression absolue comprise entre 0,5 et 10 MPa, de préférence entre 1 et 8 MPa et de manière préférée entre 2 et 6 MPa, choisie de manière à maintenir produits et réactifs sous forme liquide, et à une vitesse volumique horaire comprise entre 0,1 et 10 h⁻¹ et de préférence entre 0,4 et 5 h⁻¹.

Ladite première étape d) d'oligomérisation du procédé selon l'invention est de préférence mise en oeuvre en lit fixe. De préférence, ladite étape est réalisée dans deux réacteurs en lit fixe en série.

### Étape e) d'Oligomérisation des oléfines dite « Polynaphtha »

Conformément à l'invention, l'effluent de première oligomérisation issu de l'étape d) subit une deuxième étape e) d'oligomérisation en présence d'un catalyseur amorphe de manière à produire un effluent de deuxième oligomérisation.

La deuxième étape d'oligomérisation e) permet la production d'un effluent enrichi en oléfines ayant un nombre d'atome de carbone supérieur ou égal à 9.

Ledit effluent de deuxième oligomérisation est un effluent oléfinique comprenant avantageusement moins de 50% poids et de préférence moins de 45% poids d'oléfines ayant un nombre d'atomes de carbones compris entre 4 et 8, les pourcentages poids étant exprimés par rapport à la masse totale de l'effluent oléfinique C₄-C₈ entrant dans ladite deuxième étape d'oligomérisation e).

La conversion de l'isobutène dans l'étape e) est avantageusement supérieure à 80%, de préférence supérieure à 85%, de manière préférée supérieure à 90% et de manière très préférée supérieure à 95%.

La conversion du n-butène dans l'étape e) est avantageusement supérieure à 60%, de préférence supérieure à 70%, et de manière préférée supérieure à 80%.

La conversion des oléfines ayant un nombre d'atomes de carbone compris entre 8 et 11 dans l'étape e) est avantageusement supérieure à 40%, de préférence supérieure à 50%, et de manière préférée supérieure à 60%.

La conversion des oléfines ayant un nombre de d'atomes de carbone au moins égal à 12 dans l'étape e) est avantageusement inférieure à 5%, de préférence inférieure à 3%, et de manière préférée inférieure à 1%.

L'élévation de température dans les réacteurs due à l'exothermicité des réactions dans les étapes d) et e) sera contrôlée en faisant varier le débit de recyclage du produit léger issu de l'étape f) ainsi que la fraction du produit intermédiaire issu de l'étape f) envoyé vers l'étape g).

Les n-butènes et les oléfines ayant un nombre d'atome de carbone au moins égal à 8 étant peu réactifs dans la première étape d) d'oligomérisation, elles jouent le rôle de volant thermique dans ladite étape d).

Les oléfines ayant un nombre d'atome de carbone au moins égal à 12 étant peu réactives dans la deuxième étape e) d'oligomérisation, elles jouent le rôle de volant thermique dans ladite étape e).

Le catalyseur utilisé dans l'étape e) du procédé selon l'invention possède les mêmes caractéristiques que le catalyseur utilisé dans l'étape d) du procédé selon l'invention. De préférence, il est identique au catalyseur utilisé dans l'étape d) du procédé selon l'invention.

Ladite deuxième étape e) d'oligomérisation du procédé selon l'invention opère avantageusement à une température comprise entre 100 et 200°C, de préférence entre 110 et 160°C, à une pression absolue comprise entre 2 et 15 MPa, de préférence entre 2 et 8 MPa et de manière préférée entre 3 et 8 MPa et à une vitesse volumique horaire comprise entre 0,1 et 10 h⁻¹ et de préférence entre 0,4 et 5h⁻¹. En tout état de cause, la pression d'opération du procédé est telle que tous les réactifs et produits sont sous forme liquide dans la zone réactionnelle.

Ladite étape e) d'oligomérisation est avantageusement mise en oeuvre dans au moins un réacteur en lit fixe, préférentiellement au moins deux réacteurs en lit fixe en série, et plus préférentiellement au moins trois réacteurs en lit fixe en série.

L'opération de l'étape e) à une température élevée permet la production de molécules moins branchées que si cette étape était opérée à plus faible température, ce qui conduit à l'obtention d'un produit dont la masse volumique est plus importante.

### Étape f) de Fractionnement

Conformément à l'invention, l'effluent de deuxième oligomérisation issu de l'étape e) subit une étape de fractionnement f) dans au moins une colonne de distillation de manière à séparer ledit effluent en au moins trois produits correspondants respectivement à un produit léger comprenant majoritairement les composés C₂ à C₄, un produit intermédiaire comprenant majoritairement les composés C₅ à C₉, correspondant à une coupe essence et un produit distillat moyen comprenant majoritairement les composés ayant au moins 10 atomes de carbone dont le point de coupe est compris entre 150 et 350°C. Un produit de fond ayant un point d'ébullition initial compris entre 350 et 370 °C est également avantageusement séparé. Ces produits cités ne sont nullement restrictifs.

Ledit produit léger comprend avantageusement au moins 80% poids de n-butènes, préférentiellement 85% poids, et de manière préférée 90% poids, le pourcentage poids étant exprimé par rapport à la quantité totale de butènes présents dans ledit produit léger.

Au moins une partie dudit produit léger peut avantageusement être recyclée dans la première étape d) d'oligomérisation du procédé selon l'invention.

Par au moins une partie dudit produit léger, on entend avantageusement entre 0 et 100% poids du débit massique total dudit produit léger, préférentiellement entre 50 et 100% poids, plus préférentiellement entre 75 et 100% poids.

Au moins une partie dudit produit intermédiaire est traitée dans une étape g) d'oligomérisation.

Par au moins une partie dudit produit intermédiaire, on entend avantageusement entre 80 et 100% du débit massique total dudit produit intermédiaire, préférentiellement entre 90 et 100%, plus préférentiellement entre 95 et 100%.

Conformément à l'invention, au moins une partie dudit produit distillat moyen est traitée dans une étape h) d'hydrogénation. Par une partie dudit produit distillat moyen, on entend au moins 80% du débit total du produit distillat moyen, préférentiellement au moins 90% et de manière préférée la totalité dudit produit distillat moyen.

Ladite étape f) est avantageusement mise en oeuvre avec deux colonnes à distiller opérant en série, la première colonne à distiller fractionnant l'effluent issu de la deuxième étape e) d'oligomérisation en un produit léger comprenant majoritairement les composés C₂ à C₄ et un produit de fond de première colonne, et la seconde colonne fractionnant ledit produit de fond de première colonne en un produit intermédiaire comprenant majoritairement les composés C₅ à C₉ et un produit distillat moyen composé majoritairement des composés ayant au moins 10 atomes de carbone.

Ledit produit léger comprend avantageusement au moins 90% poids d'oléfines dont le nombre d'atomes de carbone est inférieur ou égal à 4, préférentiellement au moins 95% poids, plus préférentiellement au moins 98% poids et de manière préférée au moins 99,9% poids, ce pourcentage étant défini par rapport au poids total dudit produit léger.

Ledit produit intermédiaire comprend avantageusement au moins 90% poids d'oléfines dont le nombre d'atomes de carbone est compris entre 5 et 9, préférentiellement au moins 96% poids et plus préférentiellement au moins 99.8% poids, ce pourcentage étant défini par rapport au poids total dudit produit intermédiaire.

Ledit produit distillat moyen comprend avantageusement au moins 90% poids d'oléfines dont le nombre d'atomes de carbone est au moins égal à 10, préférentiellement au moins 95% poids, plus préférentiellement au moins 98% poids et de manière préférée, au moins 99,5% poids, ce pourcentage étant défini par rapport au poids total dudit produit distillat moyen.

### Étape g) d'Oligomérisation des oléfines C₈ dite « C₈ Polynaphtha »

Conformément à l'invention, au moins une partie dudit produit intermédiaire produit par l'étape f) du procédé selon l'invention est traitée dans une troisième étape g) d'oligomérisation.

La conversion des oléfines ayant 8 atomes de carbone dans l'étape g) est avantageusement comprise entre 20 et 40%. La sélectivité de la réaction d'oligomérisation des oléfines ayant 8 atomes de carbone vers les oléfines ayant 16 atomes de carbone est supérieure à 95%, préférentiellement supérieure à 98%, de manière préférée supérieure à 99%.

Le catalyseur utilisé dans l'étape g) du procédé selon l'invention possède les mêmes caractéristiques que le catalyseur utilisé dans l'étape d) du procédé selon l'invention. De préférence, il est identique au catalyseur utilisé dans l'étape d) du procédé selon l'invention.

Ladite étape g) d'oligomérisation du procédé selon l'invention opère avantageusement à une température comprise entre 100 et 200°C, de préférence entre 110 et 160°C, à une pression absolue comprise entre 2 et 15 MPa, de préférence entre 2 et 8 MPa et de manière préférée entre 3 et 8 MPa et à une vitesse volumique horaire comprise entre 0,1 et 5 h⁻¹ et de préférence entre 0,4 et 2 h⁻¹. En tout état de cause, la pression d'opération du procédé est telle que tous les réactifs et produits sont sous forme liquide dans la zone réactionnelle.

Ladite étape g) d'oligomérisation est avantageusement mise en oeuvre dans au moins un réacteur en lit fixe, préférentiellement au moins deux réacteurs en lit fixe en série, et plus préférentiellement au moins trois réacteurs en lit fixe en série.

### Étape h) Hydrogénation

Conformément à l'étape h) du procédé selon l'invention, au moins une partie dudit produit distillat moyen issu de l'étape f) subit une étape d'hydrogénation des oléfines en paraffines, pour les rendre incorporable au pool carburant.

De préférence, au moins une partie et de préférence la totalité dudit produit distillat moyen issu de l'étape f) est mise en contact avec un gaz riche en hydrogène en présence d'un catalyseur comprenant au moins un métal du groupe VIII, avantageusement choisi parmi le palladium et le nickel pris seul ou en mélange, et un support avantageusement choisi parmi l'alumine, la silice ou la silice-alumine.

Le catalyseur mis en oeuvre dans ladite étape h) d'hydrogénation comprend une teneur en palladium comprise entre 0,1 et 5 % poids et/ou une teneur en oxyde de nickel avantageusement comprise entre 15 et 40% poids par rapport à la masse totale du catalyseur. Ce nickel peut être promu avec du molybdène ou peut également être partiellement sulfuré. Lorsque la teneur en métal du catalyseur augmente, la température opératoire de la section réactionnelle doit être adaptée à la baisse pour compenser l'augmentation de l'activité catalytique.

L'étape h) d'hydrogénation opère avantageusement à une température comprise entre 100 et 350°C en entrée de réacteur et à une pression comprise entre 2 et 4 MPa et à une vitesse volumique horaire comprise entre 1 et 5 h⁻¹. Le ratio molaire hydrogène sur hydrocarbures H₂/HC est compris entre 10 et 450.

L'hydrogène non réagi est séparé en sortie de réacteur afin d'être recyclé en entrée. Une fraction du débit total de l'effluent de réaction débarassé de l'hydrogène est également recyclée en entrée de manière à servir de volant thermique à la réaction. Le débit de ladite fraction recyclée représente entre 2 et 5 fois le débit massique de ladite partie dudit produit distillat moyen issu de l'étape f) en entrée de l'étape h), c'est-à-dire avant le recyclage de l'hydrogène et de ladite fraction.

Les réacteurs utilisés dans l'étape h) sont des réacteurs multi-lits avec un recyclage d'une partie de l'effluent de réaction pour diluer la charge et contrôler l'exotherme. L'hydrogénation concerne quasiment exclusivement des oléfines, la réaction est donc très exothermique (entre 100 et 300°C d'exotherme)

On valide la performance de l'hydrogénation par une mesure du point de fumée et des gommes actuelles qui seront avantageusement supérieur à 25 mm pour le point de fumée et inférieure à 7mg/100ml pour la teneur en gommes actuelle. Cela se traduit généralement par une mesure du nombre de brome conformément à la norme ASTM D2710 qui est avantageusement d'au plus 10 mg Br/100g lorsque ces limites pour le point de fumée et les gommes actuelles sont respectées.

L'effluent issu de l'étape optionnelle d'hydrogénation contient majoritairement des hydrocarbures valorisables et incorporables au pool kérosène et/ou gazole et de préférence kérosène.

Le rendement en hydrocarbures dont le nombre d'atomes de carbone est au moins égal à 10 de ladite étape h) d'hydrogénation est supérieur à 90%, préférentiellement supérieur à 95%. La teneur en oléfines dans l'effluent de ladite étape h) est compris entre 0 et 5% du poids total dudit effluent, préférentiellement entre 0 et 2% poids.

Une étape de séparation optionnelle suivant l'étape h) d'hydrogénation est avantageusement mise en oeuvre pour permettre le fractionnement en une coupe kérosène et/ou une coupe gazole et/ou une coupe ayant un point d'ébullition supérieur à 360°C et/ou des coupes légères.

### EXEMPLES

### Exemple 1 (conforme)

*Cet exemple illustre l'invention.*

### Description de la charge butanol

La charge butanol utilisée dans l'exemple a été traitée par une succession d'étapes de distillation et de passages sur tamis moléculaires. Suite à ces traitements, la charge purifiée présente la composition indiquée dans le Tableau 7, colonne « charge étape a) ». La charge purifiée présente une teneur en impuretés ionique inférieure à 100 ppm, une teneur en métaux inférieure à 50 ppm, une teneur en impuretés oxygénées inférieure à 150 ppm et une teneur totale en composés azotés et soufrés inférieure à 30 ppm.

### Étape a) : déshydratation de la charge purifiée

La charge purifiée subit une étape a) de déshydratation isomérisante. Ladite étape a) est opérée à une température de 400°C, à une pression de 0,85 MPa en entrée de réacteur, et à une vitesse volumique horaire de 5 h⁻¹ en présence d'un catalyseur silice-alumine de manière à maximiser la production de butènes.

L'effluent de l'étape a), majoritairement butylènique, présente la composition indiquée dans le Tableau 7, colonne « effluent étape a) ». L'isobutanol est converti à 99,8% en un mélange de butènes proche de l'équilibre thermodynamique et en eau. La réaction produit également un certain nombre de coproduits : oxygénés et oléfines légères.

### Étape b) : séparation de l'eau de l'effluent butylènique

L'effluent majoritairement butylènique issu de l'étape a) est ensuite dirigé vers un ballon de décantation pour y effectuer la séparation de l'eau. La séparation est effectuée à 55°C et 0,75 MPa de manière à favoriser la démixtion liquide-liquide et donc la séparation des hydrocarbures de l'eau. La teneur résiduelle en eau dans l'effluent liquide organique est alors de 1800ppm.

**Tableau 1 - Répartition massique des composés hydrocarbonés dans l'effluent de l'étape b)**

| **Composé** | **composition massique (%pds des composés hydrocarbonés)** |
|---|---|
| butane | 0,4% |
| C₂ oléfine | 0,6% |
| C₃ oléfine | 0,2% |
| iC₄ oléfine | 41,5% |
| nC₄ oléfine | 56,7% |
| oléfines ayant au moins 5 atomes de carbone | 0,6% |

La fraction d'oxygénés, azotés et eau dans l'effluent liquide organique issu de l'étape b) est de 0.3%..

### Étape c) : purification de l'effluent liquide organique

L'effluent liquide organique issu de l'étape b) passe ensuite par une étape de purification afin de retirer la majeure partie de l'eau restante et les composés pouvant porter atteinte au lits catalytiques avals. Cette étape de purification permet de retirer partiellement les éléments soufrés, azotés et oxygénés restants sur un tamis moléculaire 13x, puis de retirer partiellement l'eau restante sur un tamis moléculaire 3A.

La composition de l'effluent organique purifié en sortie de l'étape c) est indiquée Tableau 2 :

**Tableau 2**

| | **Composition** (% **pds)** |
|---|---|
| oléfines | 99,92% |
| Eau | 300ppm |
| azotés , soufrés et inorganiques | <1ppm |
| composés oxygénés | 0,05% |

### Étape d) : étape d'oligomérisation sélective de l'isobutène

L'effluent organique purifié issu de l'étape c) est mélangé avec 76.5% du débit total de l'effluent de tête de l'étape f) de fractionnement ainsi qu'à tout l'effluent de l'étape g) d'oligomérisation des oléfines C₈. Ce mélange présente la composition indiquée dans le Tableau 7, colonne « charge étape d) ». Il est traité dans la première étape d'oligomérisation d), qui opère en présence du catalyseur IP811 commercialisé par Axens. Le catalyseur IP811 est un catalyseur silice-alumine amorphe. Les conditions opératoires de l'étape d) sont une température de 60°C, une pression de 3 MPa en entrée de réacteur et une vitesse volumique horaire dans les réacteurs de 2 h⁻¹ de manière à favoriser très majoritairement l'oligomérisation de l'isobutène tout en limitant la réaction des n-butènes.

L'effluent de l'étape d) d'oligomérisation présente la composition indiquée dans le Tableau 7, colonne « charge étape e) ».

À l'issue de l'étape d), à partir d'1 kg de butanol introduit dans l'étape a), 295 g d'oléfines ayant un nombre d'atomes de carbone supérieur ou égal à 5 sont produites.

### Étape e) : étape d'oligomérisation des oléfines

L'effluent de l'étape d) d'oligomérisation sélective ensuite traité dans l'étape e). Cette dernière est opérée en présence du catalyseur IP811 commercialisé par Axens. Les conditions opératoires de l'étape e) sont une température de 110°C, une pression de 6 MPa en entrée de réacteur et une vitesse volumique horaire dans les réacteurs de 2 h⁻¹.

L'effluent de l'étape e) d'oligomérisation présente la composition indiquée dans le Tableau 7, colonne « charge étape f) ».

### Étape f) : étape de fractionnement des effluents d'oligomérisation

L'effluent de l'étape e) subit ensuite une l'étape f) de fractionnement de manière à séparer un produit léger comprenant les composés C₂ à C₄, un produit intermédiaire comprenant les composés C₅ à C₉ et un produit distillat moyen, qui constituera en partie la coupe kérosène, composé des C₁₀⁺. La répartition des produits séparés est présentée Tableau 3.

**Tableau 3 - Répartition des produits à l'issue de l'étape f)**

| **Produit** | **Composition (% pds)** |
|---|---|
| Produit léger | 4,6% |
| Produit intermédiaire | 44,6% |
| Produit distillat moyen | 50,8% |

À partir d'1 kg/h de butanol en entrée de l'étape a), on obtient 63 g/h de produit léger, 618 g/h de produit intermédiaire et 702 g/h de produit distillat moyen en sortie de l'étape f). Une grande partie des deux premiers produits est recyclée pour obtenir ces résultats : 76.5% du produit léger est recyclé en entrée de l'étape d) et 96.5% du produit intermédiaire est dirigé vers l'étape g) afin d'oligomériser les oléfines en C₅-C₈ avant d'être redirigé vers l'étape d).

Le produit léger contient 500 ppm masse de composés carbonés autres que C₁-C₄. Le produit intermédiaire contient 2000 ppm masse de composés carbonés autres que C₅-C₉. Le produit distillat moyen contient 0,5% masse de composés carbonés C₁-C₉.

### Étape g) : oligomérisation des oléfines C8

96.5% du produit intermédiaire issu de l'étape f) est dirigé vers l'étape g) d'oligomérisation des oléfines C₈. Cette étape est opérée en présence du catalyseur IP811 commercialisé par Axens. Les conditions opératoires de l'étape g) sont une température de 110°C, une pression de 6 MPa en entrée de réacteur et une vitesse volumique horaire dans les réacteurs de 2 h⁻¹.

La composition de l'effluent en sortie de l'étape g) est présentée Tableau 4. Il est ensuite recyclée en entrée de l'étape d).

**Tableau 4**

| **répartition massique des composés hydrocarbonés** | **% pds** |
|---|---|
| C8= | 69,9% |
| C16= | 29,9% |
| C16+ | 0,2% |

### Étape h) : hydrogénation des oléfines

Le produit distillat moyen issu de l'étape f) de fractionnement est dirigé vers l'étape h) d'hydrogénation des oléfines qui opère avec un catalyseur LD746 commercialisé par Axens et dans laquelle la majeure partie des oléfines va être hydrogénée tout en minimisant la production de molécules hydrocarbonées légères. Cette étape opère à une température de 160°C et une pression de 2.5 MPa en entrée de réacteur. La vitesse volumique horaire dans la section réactionnelle est de 3h⁻¹ et le ratio H₂/HC est égal à 100.

L'étape h) produit un mélange d'alcanes possédant majoritairement entre 9 et 16 atomes de carbone dont la répartition est présentée Tableau 5.

**Tableau 5**

| **Composition massique de l'effluent de l'étape h) (H₂ non compris)** | **% pds** |
|---|---|
| C₁₀-C₁₆ | 78,0 |
| C₁-C₄ | 1,6 |
| C₅-C₉ | 8,2 |
| C₁₆⁺ | 12,1 |

L'effluent de l'étape h) d'hydrogénation est ensuite fractionné puis les fractions correspondantes sont mélangées avec les fractions non recyclées de produits léger et intermédiaire provenant de l'étape f).

La répartition des produits issus du procédé opéré selon l'exemple 1 est présentée Tableau 6.

**Tableau 6**

| | **% pds** |
|---|---|
| **Coupe kérosène** | 73,6% |
| **Fuel gas (C₁-C₄)** | 4,3% |
| **C₅-C₉** | 10,6% |
| **C₁₆⁺** | 11,4% |

En sortie d'étape h), à partir d'1 kg/h de butanol entrant dans l'étape a), 561 g/h de bio-kérosène sont obtenus.

**Tableau 7 - Principaux flux de l'exemple 1**

| **Description** | **Charge étape a)** | **Effluent étape a)** | **Charge étape d)** | **Charge étape e)** | **Charge étape f)** | **Charge étape g)** | **Charge étape h)** |
|---|---|---|---|---|---|---|---|
| Température (°C) | 400 | 55 | 60 | 110 | 150 | 110 | 160 |
| Pression (MPa) | 0,85 | 0,75 | 3,0 | 6,0 | 5,9 | 6,0 | 2,5 |
| Débit massique (t/h) | 16,9 | 16,9 | 23,4 | 23,4 | 23,4 | 9,9 | 11,9 |
| Composition (%poids) | | | | | | | |
| **Iso-butanol** | 69,5% | 0,1% | | | | | |
| **butane** | | 0,2% | 0,8% | 0,8% | 0,8% | 0,0% | 0,0% |
| **C2=** | | 0,3% | 0,4% | 0,4% | 0,4% | | 0,0% |
| **C3=** | | 0,1% | 0,1% | 0,1% | 0,0% | | 0,0% |
| **iC4=** | | 21,8% | 22,6% | 2,3% | | | |
| **1C4=** | | 6,2% | 33,5% | 32,5% | 3,5% | 0,2% | 0,0% |
| **T2C4=** | | 13,9% | | | | | |
| **C2C4=** | | 9,7% | | | | | |
| **C5+=** | | 0,3% | 0,3% | 0,3% | 0,3% | | 0,6% |
| **C8=** | | | 29,7% | 42,4% | 44,2% | 98,9% | 0,2% |
| **C9-C11=** | | | | | 6,8% | 0,8% | 12,7% |
| **C12=** | | | | 7,4% | 19,4% | 0,0% | 38,1% |
| **C13-C15=** | | | | | 1,7% | | 3,4% |
| **C16=** | | | 12,7% | 13,8% | 16,6% | | 32,7% |
| **C16+=** | | | | | 6,2% | | 12,3% |
| **H2O** | 29,5% | 47,3% | | | | | |
| **Autres alcools** | 1% | | | | | | |

### Exemple 2 (non conforme) - Procédé de production sans oligomérisation du produit intermédiaire recyclé

*Dans cet exemple, le produit intermédiaire issu de l*'*étape f) est recyclé en entrée de étape d) sans oligomérisation dans une étape g).*

La charge, ainsi que les étapes a) et b) et c) sont identiques à l'exemple 1.

### Étape d) : étape d'oligomérisation de l'isobutène

L'effluent majoritairement butylènique issu de l'étape c) est ensuite mélangé à 77% du produit léger et 87.5% du produit intermédiaire issus de l'étape f) de fractionnement puis envoyé dans la première étape d'oligomérisation d), laquelle est opérée dans les mêmes conditions opératoires et avec le même catalyseur que dans l'exemple 1.

La composition de l'effluent en sortie de réaction est présentée Tableau 8.

**Tableau 8**

| **Composition massique de l'effluent en sortie de l'étape d)** | **% pds** |
|---|---|
| butane | 0,6% |
| C2= | 0,4% |
| C3= | 0,1% |
| iC4= | 2,2% |
| nC4= | 31,3% |
| C5+= | 0,3% |
| C8= | 56,9% |
| C12= | 7,2% |
| C16= | 1,0% |

A l'issue de cette étape, à partir d'1 kg/h de butanol entrant dans l'étape a), 295 g/h d'oléfines ayant plus de 5 atomes de carbones sont produites.

L'effluent de l'étape d) est ensuite dirigé vers l'étape e) d'oligomérisation des oléfines.

### Étape e) : étape d'oligomérisation des oléfines

L'étape e) opère dans les mêmes conditions et avec le même catalyseur que l'exemple 1. La composition de l'effluent de deuxième oligomérisation est indiquée Tableau 9.

**Tableau 9**

| **répartition massique des composés hydrocarbonés** | **% pds** |
|---|---|
| butane | 0,6% |
| C2= | 0,4% |
| nC4= | 3,4% |
| C5+= | 0,3% |
| C8= | 51,0% |
| C9-C11= | 6,4% |
| C12= | 23,9% |
| C13-C15= | 1,9% |
| C16= | 5,5% |
| C16+= | 6,4% |

### Étape f) : étape de fractionnement des effluents d'oligomérisation

L'effluent de l'étape e) est ensuite fractionné en 3 produits dans l'étape f), de la même manière que dans l'exemple 1. La répartition des produits séparés est présentée Tableau 10.

**Tableau 10**

| **Produit** | **% pds** |
|---|---|
| Produit léger | 4,3% |
| Produit intermédiaire | 51,4% |
| Produit distillat moyen | 44,3% |

A partir d'1 kg/h de butanol en entrée de l'étape a) on obtient : 61 g/h de produit léger, 733g/h produit intermédiaire et 633g/h de produit distillat moyen. 77% masse du produit léger et 87.5% masse du produit intermédiaire sont recyclés en entrée en l'étape d).

Le produit léger contient 500 ppm masse de composés carbonés autres que C₁-C₄. Le produit intermédiaire contient 2000 ppm masse de composés carbonés autres que C₅-C₉. Le produit distillat moyen contient 0,5% masse de composés carbonés C₁-C₉.

### Étape h) : hydrogénation des oléfines

Le produit distillat moyen issu de l'étape f) de fractionnement est dirigé vers l'étape h) d'hydrogénation des oléfines. L'étape h) opère dans les mêmes conditions et avec le même catalyseur que l'exemple 1. L'effluent de l'hydrogénation a la composition présentée dans le Tableau 11.

**Tableau 11**

| **Composition massique de l'effluent de l'étape h) (H₂ non compris)** | **% pds** |
|---|---|
| C₁₀-C₁₆ | 66,2% |
| C₁-C₄ | 1,4% |
| C₅-C₉ | 19,9% |
| C₁₆⁺ | 12,5% |

L'effluent de l'étape h) d'hydrogénation est ensuite fractionné puis les fractions correspondantes sont mélangées avec les fractions non recyclées de produits léger et intermédiaire provenant de l'étape f).

La répartition des produits issus du procédé opéré selon l'exemple 2 est présentée Tableau 12.

**Tableau 12**

| | **% pds** |
|---|---|
| **Coupe kérosène** | 64,1% |
| **Fuel gas (C₁-C₄)** | 4,5% |
| **C₅-C₉** | 19,3% |
| **C₁₆⁺** | 12,1% |

En sortie d'étape h), à partir d'1 kg/h de butanol entrant dans l'étape a), 490g de bio-kérosène sont obtenus.

### Exemple 3 (non conforme) - Procédé de production sans recyclage du produit intermédiaire

*Dans cet exemple, ni le produit intermédiaire ni le produit léger provenant de l'étape f) de fractionnement ne sont recyclés en entrée de l'étape d) d'oligomérisation. En revanche, le produit intermédiaire subit une étape g) d'oligomérisation afin d'améliorer le rendement de réaction.*

La charge, ainsi que les étapes a), b) et c) sont identiques à l'exemple 1.

### Étape d) : étape d'oligomérisation de l'isobutène

L'effluent majoritairement butylènique issu de l'étape c) est ensuite envoyé dans la première étape d'oligomérisation d), opérée dans les mêmes conditions opératoires et avec le même catalyseur que dans l'exemple 1. La composition de l'effluent en sortie de réaction est présentée Tableau 13.

**Tableau 13**

| **répartition massique des composés hydrocarbonés** | **% pds** |
|---|---|
| butane | 0,4% |
| C2= | 0,7% |
| C3= | 0,1% |
| iC4= | 4,2% |
| nC4= | 55,1% |
| C5+= | 0,5% |
| C8= | 23,4% |
| C12= | 13,6% |
| C16= | 1,9% |

A l'issue de cette étape, à partir d'1 kg/h de butanol entrant dans l'étape a), 294 g/h d'oléfines ayant plus de 5 atomes de carbones sont produites.

L'effluent de l'étape d) est ensuite dirigé vers l'étape e) d'oligomérisation des oléfines.

### Étape e) : étape d'oligomérisation des oléfines

L'étape e) opère dans les mêmes conditions et avec le même catalyseur que l'exemple 1. La composition de l'effluent oléfinique de l'étape e) est présentée Tableau 14.

**Tableau 14**

| **répartition massique des composés hydrocarbonés** | **% pds** |
|---|---|
| butane | 0,4% |
| C2= | 0,7% |
| iC4= | 5,9% |
| C5+= | 0,6% |
| C8= | 39,0% |
| C9-C11= | 6,7% |
| C12= | 31,2% |
| C13-C15= | 2,0% |
| C16= | 6,7% |
| C16+= | 6,7% |

### Étape f) : étape de fractionnement des effluents d'oligomérisation

L'effluent de l'étape e) est ensuite fractionné en 3 produits dans l'étape f), de la même manière que dans l'exemple 1. La répartition des produits séparés est présentée Tableau 15.

**Tableau 15**

| **Produit** | **% pds** |
|---|---|
| Produit léger | 7,0% |
| Produit intermédiaire | 39,5% |
| Produit distillat moyen | 53,5% |

A partir d'1 kg/h de butanol en entrée de l'étape a) on obtient : 52g/h de produit léger, 295g/h de produit intermédiaire et 400g/h de produit distillat moyen. Aucun produit n'est recyclé dans cet exemple, contrairement à ce qui est fait dans les exemples 1 et 2. La totalité du produit intermédiaire est dirigé vers l'étape g) d'oligomérisation des oléfines C₈ afin d'augmenter le rendement final. Le produit léger est intégré tel quel dans les produits. Le produit distillat moyen est dirigé vers l'étape d'hydrogénation h).

Le produit léger contient 500 ppm masse de composés carbonés autres que C₁-C₄. Le produit intermédiaire contient 2000 ppm masse de composés carbonés autres que C₅-C₉. Le produit distillat moyen contient 0,5% masse de composés carbonés C₁-C₉.

### Étape g) : oligomérisation des oléfines C₈

Le produit intermédiaire issu de l'étape f) de fractionnement est dirigé vers l'étape g). L'étape g) opère dans les mêmes conditions et avec le même catalyseur que dans l'exemple 1. La composition de l'effluent en sortie de l'étape g) est indiquée Tableau 16.

**Tableau 16**

| **répartition massique des composés hydrocarbonés** | **% pds** |
|---|---|
| C8= | 69,9% |
| C16= | 29,9% |
| C16+ | 0,2% |

Il est ensuite dirigé vers l'étape h) d'hydrogénation des oléfines pour réduire sa teneur en oléfines conjointement au produit distillat moyen issu de l'étape f) de fractionnement.

### Étape h) : hydrogénation des oléfines

Le produit distillat moyen issu de l'étape f) de fractionnement et l'effluent de l'étape g) sont dirigés vers l'étape h) d'hydrogénation des oléfines. L'étape h) opère dans les mêmes conditions et avec le même catalyseur que l'exemple 1. L'effluent de l'hydrogénation a la composition indiquée dans le Tableau 17.

**Tableau 17**

| **Composition massique de l'effluent de l'étape h) (H₂ non compris)** | **% pds** |
|---|---|
| C₁₀-C₁₆ | 57,0% |
| C₁-C₄ | 1,2% |
| C₅-C₉ | 34,6% |
| C₁₆⁺ | 7,3% |

L'effluent de l'étape h) d'hydrogénation est ensuite fractionné puis les fractions correspondantes sont mélangées avec les fractions de produits léger et intermédiaire provenant de l'étape f).

La composition de sortie de l'effluent hydrocarboné du procédé est indiquée Tableau 18.

**Tableau 18**

| | **% pds** |
|---|---|
| **Coupe kérosène** | 52,9% |
| **Fuel gas (C₁-C₄)** | 8,3% |
| **C₅-C₉** | 32,1% |
| **C₁₆⁺** | 6,7% |

En sortie d'étape h), à partir d'1 kg/h de butanol entrant dans l'étape a), 402 g de bio-kérosène sont obtenus.

### Exemple 4 (non conforme) - Procédé de production sans recyclage du produit intermédiaire ni oligomérisation dudit produit intermédiaire

*Cet exemple est similaire à l'exemple 3. Cependant, le produit intermédiaire ne subit pas d'étape g) d'oligomérisation avant d'être incorporé au pool carburant.*

La charge, ainsi que les étapes a) à f) sont identiques à l'exemple 3.

Le produit distillat moyen issu de l'étape f) est hydrogéné dans l'étape h) d'hydrogénation des oléfines. Les produits léger et intermédiaire issus de l'étape f) sont directement intégrées dans les produits du procédé.

### Étape h) : hydrogénation des oléfines

Le produit distillat moyen issu de l'étape f) de fractionnement est dirigé vers l'étape h) d'hydrogénation des oléfines. L'étape h) opère dans les mêmes conditions et avec le même catalyseur que dans l'exemple 1.

L'effluent de l'hydrogénation a la composition suivante présentée dans le Tableau 19.

**Tableau 19**

| **Composition massique de l'effluent de l'étape g) (H2 non compris)** | **% pds** |
|---|---|
| C₁₀-C₁₆ | 77,7% |
| C1-C4 | 1,6% |
| C5-C9 | 8,2% |
| C16+ | 12,5% |

L'effluent de l'étape h) d'hydrogénation est ensuite fractionné puis les fractions correspondantes sont mélangées avec les fractions de produits léger et intermédiaire provenant de l'étape f).

La composition de sortie de l'effluent hydrocarboné du procédé est indiquée Tableau 20.

**Tableau 20**

| | **% pds** |
|---|---|
| **Coupe kérosène** | 41,8% |
| **Fuel gas (C₁-C₄)** | 8,1% |
| **C₅-C₉** | 43,4% |
| **C₁₆⁺** | 6,7% |

En sortie d'étape h), à partir d'1 kg/h de butanol entrant dans l'étape a), 319 g de bio-kérosène sont obtenus.

### Exemple 5 (non conforme) - Procédé de production sans oligomérisation sélective des isobutènes, avec oligomérisation du produit intermédiaire et recyclage

*Dans cet exemple, on réalise l'oligomérisation des butènes en une seule étape et non en deux étapes d) et e) comme enseigné dans l'invention. Les i-butènes, plus réactifs, réagissent alors en même temps que les autres oléfines comprenant de 4 à 8 atomes de carbone.*

La charge, ainsi que les étapes a), b) et c) sont identiques à l'exemple 1.

### Étape d'oligomérisation des oléfines

L'effluent majoritairement butylènique issu de l'étape c) est mélangé à 35.7% du produit léger issu de l'étape f) ainsi qu'à la totalité de l'effluent de l'étape g) puis envoyé dans l'étape d'oligomérisation, opérée en présence du catalyseur IP811 commercialisé par Axens, à une température de 60°C, une pression de 3 MPa en entrée de réacteur et une vitesse volumique horaire de 2 h⁻¹. La composition de l'effluent oléfinique de l'étape d'oligomérisation est indiquée Tableau 21.

**Tableau 21**

| **répartition massique des composés hydrocarbonés** | **% pds** |
|---|---|
| butane | 0,9% |
| C2= | 0,4% |
| iC4= | 5,7% |
| C5+= | 0,3% |
| C8= | 41,9% |
| C9-C11= | 6,9% |
| C12= | 17,8% |
| C13-C15= | 2,1% |
| C16= | 17,3% |
| C16+= | 6,9% |

A l'issue de cette étape, à partir d'1 kg/h de butanol entrant dans l'étape a), 937 g/h d'oléfines ayant plus de 5 atomes de carbones sont produites.

### Étape f) : étape de fractionnement des effluents d'oligomérisation

L'effluent de l'étape d'oligomérisation est ensuite fractionné en 3 produits dans l'étape f), de la même manière que dans l'exemple 1. La répartition des produits séparés est présentée Tableau 22.

**Tableau 22**

| **Produit** | % **pds** |
|---|---|
| Produit léger | 6,8% |
| Produit intermédiaire | 42,2% |
| Produit distillat moyen | 51,0% |

A partir d'1 kg/h de butanol en entrée de l'étape a) on obtient : 93 g/h de produit léger, 576 g/h de produit intermédiaire et 695 g/h de produit distillat moyen.

35.7% du produit léger est recyclé en amont de l'étape d'oligomérisation et 100% du produit intermédiaire est dirigé vers l'étape g) afin d'oligomériser les oléfines en C₅-C₈ avant d'être recyclé vers l'étape d'oligomérisation.

Le produit léger contient 500 ppm masse de composés carbonés autres que C₁-C₄. Le produit intermédiaire contient 2000 ppm masse de composés carbonés autres que C₅-C₉. Le produit distillat moyen contient 0,5% masse de composés carbonés C₁-C₉.

### Étape g) : oligomérisation des oléfines C8=

Le produit intermédiaire issu de l'étape f) de fractionnement est dirigé vers l'étape g) d'oligomérisation des oléfines C₈. L'étape g) opère dans les mêmes conditions et avec le même catalyseur que dans l'exemple 1. La composition de l'effluent en sortie de l'étape g) est indiquée Tableau 23.

**Tableau 23**

| **répartition massique des composés hydrocarbonés** | **% pds** |
|---|---|
| C8= | 69,9% |
| C16= | 29,9% |
| C16=+ | 0,2% |

L'effluent de l'étape g) est ensuite recyclée vers l'étape d'oligomérisation.

### Étape h) : hydrogénation des oléfines

Le produit distillat moyen issu de l'étape f) de fractionnement est dirigé vers l'étape h) d'hydrogénation des oléfines. L'étape h) opère dans les mêmes conditions et avec le même catalyseur que dans l'exemple 1. L'effluent de l'hydrogénation a la composition indiquée dans le Tableau 24.

**Tableau 24**

| **Composition massique de l'effluent de l'étape h) (H₂ non compris)** | **% pds** |
|---|---|
| C₁₀-C₁₆ | 77,3% |
| C₁-C₄ | 1,6% |
| C₅-C₉ | 7,8% |
| C₁₆⁺ | 13,3% |

L'effluent de l'étape h) d'hydrogénation est ensuite fractionné puis les fractions correspondantes sont mélangées avec les fractions de produits léger et intermédiaire non recyclées provenant de l'étape f).

La composition de sortie de l'effluent hydrocarboné du procédé est indiquée Tableau 25.

**Tableau 25**

| | **% pds** |
|---|---|
| **Coupe kérosène** | **71,8%** |
| **Fuel gas (C₁-C₄)** | 8,6% |
| **C₅-C₉** | 7,3% |
| **C₁₆⁺** | 12,4% |

En sortie d'étape h), à partir d'1 kg/h de butanol entrant dans l'étape a), 547g de bio-kérosène sont obtenus.

### Synthèse des résultats des exemples 1 à 5

Le Tableau 26 compare quelques résultats clefs des exemples 1 à 5. Le rendement bio-kérosène correspond à la quantité de bio-kérosène produite pour 1 kg de butanol dans la charge du procédé.

**Tableau 26**

| | **Exemple 1 Conforme Cas de base** | **Exemple 2 Sans étape g)** | **Exemple 3 Pas de recyclage - oligomérisation du produit intermédiaire** | **Exemple 4 Pas de recyclage - pas d'oligomérisatio n du produit intermédiaire** | **Exemple 5 Une seule étape d'oligomérisa tion** |
|---|---|---|---|---|---|
| **% butènes convertis** | 98,37% | 98,10% | 93,96% | 93,96% | 94,05% |
| **ΔT étape d)** | 60,2°C | 57,0°C | 94,3°C | 94,3°C | - |
| **ΔT étape e)** | 141,0°C | 144,0°C | 203,0°C | 203,0°C | 196.0°C |
| **Rendement bio-kérosène*** | 56,15% | 48,98% | 40,24% | 31,86% | 54,76% |

| | | | | | |
|---|---|---|---|---|---|
| *Les rendements incluent une perte de 5% dans l'hydrogénation de la coupe kérosène (étape h). | | | | | |

L'exemple 1 montre que l'exotherme dans les deux étapes d) et e) est nettement moins important et le rendement kérosène plus élevé que dans un cas sans recyclage ni oligomérisation du produit intermédiaire (exemples 3 et 4). L'exemple 2 montre que l'ajout de l'étape g) permet d'améliorer le rendement kérosène.

L'exemple 5, avec une seule étape d'oligomérisation, mais comportant le recyclage des produits léger et intermédiaires et l'oligomérisation de ces derniers présente un rendement équivalent à l'exemple 1 conforme. Cependant, le pourcentage de butènes convertis est plus faible et le contrôle de la réaction et les produits obtenus sont différents. En effet, la séparation en deux étapes de l'oligomérisation (étapes d et e) permet de n'effectuer que les réactions voulues dans chaque étape et ainsi de contrôler à la fois la qualité des produits obtenus et les exothermes dans chaque étape. La maitrise des exothermes dans chaque étape permet également d'augmenter la durée de vie du catalyseur (stress thermique). Cet impact concerne également la sécurité (baisse de l'impact d'un à-coup sur le changement de la composition de la charge). La flexibilité est donc plus grande.

### Exemple 6 - Ajouts d'aromatiques et effet sur la densité

Le produit kérosène obtenu dans l'exemple 1 conforme a une densité d'environ 780 kg/m³. On ajoute à ce produit du Mesitylène (1,3,5-triméthylbenzène) dont la densité est de 865 kg/m³.

L'ajout de Mesitylène dans notre kérosène jusqu'à une teneur de 8%vol. monte la densité du mélange à 787kg/m3.

L'ajout de Mesitylène dans notre kérosène jusqu'à une teneur de 25%vol. monte la densité à 801 kg/m3.

On reste donc toujours dans les limites de densité fixées par la norme ASTM D7566-11a, même en ajoutant des aromatiques jusqu'aux limites prévues par ladite norme.

## Revendications

1. Procédé de production de bases hydrocarbonées distillats moyens à partir d'une charge butanol, ledit procédé comprenant au moins :
a) une étape de déshydratation isomérisante de ladite charge butanol en présence d'un catalyseur acide amorphe ou zéolithique dans au moins un réacteur, opérant à une pression absolue en entrée de réacteur comprise entre 0,5 et 1,2 MPa et à une température en entrée de réacteur comprise entre 350 et 450°C de manière à produire un effluent majoritairement butylènique,
b) une étape de séparation de l'eau présente dans ledit effluent butylènique opérant à une pression comprise entre 0,5 et 1,2 MPa et à une température comprise entre 35 et 60°C,
c) une étape de purification de l'effluent liquide organique issu de l'étape b) de manière à produire un effluent organique purifié,
d) une première étape d'oligomérisation d'une charge comprenant au moins une partie de l'effluent organique purifié issu de l'étape c), la totalité de l'effluent issu de l'étape g) et au moins une partie du produit léger issu de l'étape f) en présence d'un catalyseur amorphe dans au moins un réacteur opérant à une pression absolue comprise entre 0,5 et 10 MPa, à une température comprise entre 40 et 110°C et à une vitesse volumique horaire comprise entre 0,1 et 10 h⁻¹, de manière à produire un effluent de première oligomérisation, comprenant au moins 50% poids d'oléfines ayant un nombre d'atomes de carbone supérieur ou égal à 8, le pourcentage poids étant exprimé par rapport à la masse totale des oléfines contenues dans ledit effluent,
e) une deuxième étape d'oligomérisation dudit effluent de première oligomérisation, en présence d'un catalyseur amorphe dans au moins un réacteur opérant à une pression absolue comprise entre 2 et 15 MPa, à une température comprise entre 100 et 200°C et à une vitesse volumique horaire comprise entre 0,1 et 10 h⁻¹, de manière à produire un effluent de deuxième oligomérisation,
f) une étape de fractionnement dudit effluent de deuxième oligomérisation en au moins trois produits correspondants respectivement à un produit léger comprenant majoritairement les composés C₂ à C₄, un produit intermédiaire comprenant majoritairement les composés C₅ à C₉, et un produit distillat moyen comprenant majoritairement les composés ayant au moins 10 atomes de carbone,
g) une troisième étape d'oligomérisation d'au moins une partie dudit produit intermédiaire en présence d'un catalyseur amorphe dans au moins un réacteur opérant à une pression absolue comprise entre 2 et 15 MPa, à une température comprise entre 100 et 200°C et à une vitesse volumique horaire comprise entre 0,1 et 5 h⁻¹,
h) une étape d'hydrogénation d'au moins une partie dudit produit distillat moyen en présence d'un catalyseur comprenant au moins un métal du groupe VIII dans au moins un réacteur opérant à une pression absolue comprise entre 2 et 4 MPa, à une température comprise entre 100 et 350°C et à une vitesse volumique horaire comprise entre 1 et 5 h⁻¹ avec un ratio molaire hydrogène sur hydrocarbures H₂/HC compris entre 10 et 450.

2. Procédé selon la revendication 1 dans lequel une étape de purification de ladite charge butanol est effectuée préalablement à l'étape a).

3. Procédé selon d'une des revendications 1 à 2 dans lequel le catalyseur utilisé dans l'étape a) de déshydratation est un catalyseur zéolithique comprenant au moins une zéolithe choisie parmi les zéolithes ayant au moins des ouvertures de pores contenant 10 ou 12 atomes d'oxygènes.

4. Procédé selon d'une des revendications 1 à 2 dans lequel le catalyseur utilisé dans l'étape a) de déshydratation est un catalyseur acide amorphe comprenant au moins un oxyde réfractaire poreux choisi parmi l'alumine, l'alumine activée par un dépôt d'acide minéral et la silice alumine.

5. Procédé selon l'une des revendications 1 à 4 dans lequel Ladite étape b) est une étape de décantation dans laquelle une phase aqueuse est séparée d'une phase organique.

6. Procédé selon l'une des revendications 1 à 5 dans lequel entre 80 et 100% du débit massique total dudit produit intermédiaire est traitée dans ladite étape g) d'oligomérisation.

7. Procédé selon l'une des revendications 1 à 6 dans lequel au moins 80% du débit total du produit distillat moyen est traitée dans ladite étape h) d'hydrogénation.

8. Procédé selon l'une des revendications 1 à 7 dans lequel ladite étape f) est mise en oeuvre avec deux colonnes à distiller opérant en série.

9. Procédé selon l'une des revendications 1 à 8 dans lequel une étape de séparation suivant l'étape h) d'hydrogénation est mise en oeuvre pour permettre le fractionnement en une coupe kérosène et/ou une coupe gazole et/ou une coupe ayant un point d'ébullition supérieur à 360°C et/ou des coupes légères.

## Patentansprüche

1. Verfahren zur Herstellung von kohlenwasserstoffhaltigen Ausgangsprodukten für Mitteldestillate aus einer Butanolbeschickung, wobei das Verfahren mindestens Folgendes umfasst:
a) einen Schritt zur isomerisierenden Dehydrierung der Butanolbeschickung in Gegenwart eines amorphen oder zeolithischen sauren Katalysators in mindestens einem Reaktor, der bei einem absoluten Druck am Eingang des Reaktors im Bereich zwischen 0,5 und 1,2 MPa und bei einer Temperatur am Eingang des Reaktors im Bereich zwischen 350 und 450 °C arbeitet, um einen überwiegend Butylen enthaltenden Abfluss zu erzeugen,
b) einen Schritt zum Abtrennen des Wassers, das in dem Butylen enthaltenden Abfluss vorhanden ist, der bei einem Druck im Bereich zwischen 0,5 und 1,2 MPa und bei einer Temperatur im Bereich zwischen 35 und 60 °C arbeitet,
c) einen Schritt zum Reinigen des organischen flüssigen Abflusses, der aus Schritt b) stammt, um einen gereinigten organischen Abfluss zu erzeugen,
d) einen ersten Schritt zur Oligomerisierung einer Beschickung, umfassend mindestens einen Teil des aus Schritt c) stammenden gereinigten organischen Abflusses, wobei die Gesamtheit des aus schritt g) stammenden Abflusses und mindestens ein Teil des aus Schritt f) stammenden leichten Produkts in Gegenwart eines amorphen Katalysators in mindestens einem Reaktor, der bei einem absoluten Druck im Bereich zwischen 0,5 und 10 MPa, bei einer Temperatur im Bereich zwischen 40 und 110 °C und bei einer Raumgeschwindigkeit pro Stunde im Bereich zwischen 0,1 und 10 h⁻¹ arbeitet, um einen Abfluss aus der ersten Oligomerisierung zu erzeugen, der mindestens 50 Gew.-% Olefine umfasst, die eine Anzahl an Kohlenstoffatomen größer oder gleich 8 aufweisen, wobei der Gewichtsanteil in Bezug auf das Gesamtgewicht der Olefine ausgedrückt wird, die in dem Abfluss enthalten sind,
e) einen zweiten Schritt zur Oligomerisierung des Abflusses aus der ersten Oligomerisierung in Gegenwart eines amorphen Katalysator in mindestens einem Reaktor, der bei einem absoluten Druck im Bereich zwischen 2 und 15 MPa, bei einer Temperatur im Bereich zwischen 100 und 200 °C und bei einer Raumgeschwindigkeit pro Stunde im Bereich zwischen 0,1 und 10 h⁻¹ arbeitet, um einen Abfluss der zweiten Oligomerisierung zu erzeugen,
f) einen Schritt zur Fraktionierung des Abflusses aus der zweiten Oligomerisierung in mindestens drei Produkte, die jeweils einem leichten Produkt, umfassend überwiegend die Verbindungen C₂ bis C₄, einem Zwischenprodukt, umfassend überwiegend die Verbindungen C₅ bis C₉, und ein Mitteldestillat-Produkt, umfassend überwiegend die Verbindungen, die mindestens 10 Kohlenstoffatome aufweisen, entsprechen,
g) einen dritten Schritt zur Oligomerisierung mindestens eines Teils des Zwischenprodukts in Gegenwart eines amorphen Katalysators in mindestens einem Reaktor, der bei einem absoluten Druck im Bereich zwischen 2 und 15 MPa, bei einer Temperatur im Bereich zwischen 100 und 200 °C und bei einer Raumgeschwindigkeit pro Stunde im Bereich zwischen 0,1 und 5 h⁻¹ arbeitet,
h) einen Schritt zur Hydrierung mindestens eines Teils des Mitteldestillat-Produkts in Gegenwart eines Katalysators, umfassend mindestens ein Metall der Gruppe VIII in mindestens einem Reaktor, der bei einem absoluten Druck im Bereich zwischen 2 und 4 MPa, bei einer Temperatur im Bereich zwischen 100 und 350 °C und bei einer Raumgeschwindigkeit pro Stunde im Bereich zwischen 1 und 5 h⁻¹ mit einem Molverhältnis Wasserstoff zu Kohlenwasserstoffe H₂/HC im Bereich zwischen 10 und 450 arbeitet.

2. Verfahren nach Anspruch 1, wobei ein Schritt zum Reinigen der Butanolbeschickung vor Schritt a) durchgeführt wird.

3. Verfahren nach einem der Ansprüche 1 bis 2, wobei der in Schritt a) zum Dehydrieren verwendete Katalysator ein zeolithischer Katalysator ist, der mindestens einen Zeolithen umfasst, ausgewählt aus den Zeolithen, die mindestens Porenöffnungen aufweisen, die 10 oder 12 Sauerstoffatome enthalten.

4. Verfahren nach einem der Ansprüche 1 bis 2, wobei der in Schritt a) zur Dehydrierung verwendete Katalysator ein amorpher, sauerer Katalysator ist, der mindestens ein poröses feuerfestes Oxid, ausgewählt aus Aluminiumoxid, Aluminiumoxid, das durch eine Abscheidung einer mineralischen Säure aktiviert wurde, und Siliciumdioxid-Aluminiumoxid umfasst.

5. Verfahren nach einem der Ansprüche 1 bis 4, wobei der Schritt b) ein Schritt zum Absetzen ist, in dem eine wässrige Phase von einer organischen Phase getrennt wird.

6. Verfahren nach einem der Ansprüche 1 bis 5, wobei zwischen 80 und 100 % des Gesamtmassendurchsatzes des Zwischenprodukts in Schritt g) zur Oligomerisierung behandelt wird.

7. Verfahren nach einem der Ansprüche 1 bis 6, wobei mindestens 80 % des Gesamtmassendurchsatzes des Mitteldestillatprodukts in Schritt h) zur Hydrierung behandelt wird.

8. Verfahren nach einem der Ansprüche 1 bis 7, wobei Schritt f) mit zwei Destillationskolonnen durchgeführt wird, die in Reihe arbeiten.

9. Verfahren nach einem der Ansprüche 1 bis 8, wobei ein Schritt zur Trennung nach Schritt h) zur Hydrierung durchgeführt wird, um die Fraktionierung in einen Kerosinschnitt und/oder einen Gasölschnitt und/oder einen Schnitt, der einen Siedepunkt größer 360 °C aufweist, und/oder leichte Schnitte zu ermöglichen.

## Claims

1. Process for the production of middle-distillate hydrocarbon-containing bases from a butanol feedstock, with said process comprising at least:
a) A stage for isomerizing dehydration of said butanol feedstock in the presence of an amorphous or zeolitic acid catalyst in at least one reactor, operating at an absolute pressure at the reactor inlet of between 0.5 and 1.2 MPa and at a temperature at the reactor inlet of between 350 and 450°C in such a way as to produce an effluent that is for the most part butylenic,
b) A stage for separation of the water that is present in said butylenic effluent that operates at a pressure of between 0.5 and 1.2 MPa and at a temperature of between 35 and 60°C,
c) A stage for purification of the organic liquid effluent that comes from stage b) in such a way as to produce a purified organic effluent,
d) A first stage for oligomerization of a feedstock that comprises at least a portion of the purified organic effluent that comes from stage c), the entire effluent that comes from stage g), and at least a portion of the light product that comes from stage f) in the presence of an amorphous catalyst in at least one reactor that operates at an absolute pressure of between 0.5 and 10 MPa, at a temperature of between 40 and 110°C, and at an hourly volumetric flow rate of between 0.1 and 10 h⁻¹, in such a way as to produce a first oligomerization effluent, comprising at least 50% by weight of olefins having a number of carbon atoms that is greater than or equal to 8, with the percentage by weight being expressed relative to the total mass of olefins contained in said effluent,
e) A second stage for oligomerization of said first oligomerization effluent, in the presence of an amorphous catalyst in at least one reactor that operates at an absolute pressure of between 2 and 15 MPa, at a temperature of between 100 and 200°C, and at an hourly volumetric flow rate of between 0.1 and 10 h⁻¹, in such a way as to produce a second oligomerization effluent,
f) A stage for fractionation of said second oligomerization effluent into at least three products that correspond respectively to a light product that for the most part comprises the C₂ to C₄ compounds, an intermediate product that for the most part comprises the C₅ to C₉ compounds, and a middle distillate product that for the most part comprises the compounds that have at least 10 carbon atoms,
g) A third stage for oligomerization of at least a portion of said intermediate product in the presence of an amorphous catalyst in at least one reactor that operates at an absolute pressure of between 2 and 15 MPa, at a temperature of between 100 and 200°C, and at an hourly volumetric flow rate of between 0.1 and 5 h⁻¹, and
h) A stage for hydrogenation of at least a portion of said middle distillate product in the presence of a catalyst that comprises at least one metal of group VIII in at least one reactor that operates at an absolute pressure of between 2 and 4 MPa, at a temperature of between 100 and 350°C, and at an hourly volumetric flow rate of between 1 and 5 h⁻¹ with a hydrogen to hydrocarbon H_{2/}HC molar ratio of between 10 and 450.

2. Process according to Claim 1, in which a stage for purification of said butanol feedstock is carried out prior to stage a).

3. Process according to one of Claims 1 to 2, in which the catalyst that is used in the dehydration stage a) is a zeolitic catalyst that comprises at least one zeolite that is selected from among the zeolites that have at least pore openings containing 10 or 12 oxygen atoms.

4. Process according to one of Claims 1 to 2, in which the catalyst that is used in the dehydration stage a) is an amorphous acid catalyst that comprises at least one porous refractory oxide that is selected from among alumina, alumina activated by a deposit of mineral acid and silica-alumina.

5. Process according to one of Claims 1 to 4, in which said stage b) is a decanting stage in which an aqueous phase is separated from an organic phase.

6. Process according to one of Claims 1 to 5, in which between 80 and 100% of the total mass flow rate of said intermediate product is treated in said oligomerization stage g).

7. Process according to one of Claims 1 to 6, in which at least 80% of the total flow of the middle distillate product is treated in said hydrogenation stage h).

8. Process according to one of Claims 1 to 7, in which said stage f) is implemented with two distillation columns operating in series.

9. Process according to one of Claims 1 to 8, in which a separation stage following the hydrogenation stage h) is implemented for making possible the fractionation into a kerosene fraction and/or a diesel fuel fraction and/or a fraction that has a boiling point of higher than 360°C and/or light fractions.
